# EUROPEAN PATENT SPECIFICATION

(11) **EP 3 485 825 B1**
(45) Date of publication and mention of the grant of the patent: **28.08.2024**
(21) Application number: 16908490.2
(22) Date of filing: 15.07.2016
(51) Int. Cl.: A61B 17/34, A61B 17/00

(54) **NEEDLE GROOVE BODY, PUNCTURE FRAME BODY AND PUNCTURE FRAME**
NADELRILLENKÖRPER, PUNKTIONSRAHMENKÖRPER UND PUNKTIONSRAHMEN
CORPS DE RAINURES D'AIGUILLES ET CADRE DE PERFORATION.

(43) Date of publication of application: 22.05.2019
(73) Proprietor: Wang, Qin, Suzhou, Jiangsu 215104 (CN)
(72) Inventor: Wang, Qin, Suzhou, Jiangsu 215104 (CN)
(74) Representative: Tiburzi, Andrea
(86) International application number: PCT/CN2016/090134
(87) International publication number: WO 2018/010165

(56) References cited:
- WO-A1-2015/100332
- WO-A1-2015/159129
- CN-A- 102 283 694
- CN-A- 104 173 107
- CN-U- 202 397 562
- CN-Y- 201 394 057
- JP-A- 2001 087 264
- US-A1- 2005 143 753
- US-A1- 2007 078 346
- US-A1- 2007 276 253
- US-A1- 2009 143 684

## Description

### Technical Field

The present invention relates to the technical field ofultrasonic B-ultrasound puncture operation tools, and specifically relates to a needle groove body, a puncture frame body and a puncture frame.

### Background Art

The current *in vitro* puncture frames generally control the needle insertion in two ways: 1. using a rotating needle groove structure; and 2. using a single needle groove body structure, wherein individual needle groove bodies all have thicknesses of different specifications for one-to-one correspondence to puncture needles, so each puncture frame body using the single needle groove body structure must be equipped with needle groove bodies of various specifications.In B-ultrasound puncture operations, the doctor would select, according to the clinical use, needle groove bodies of different specifications for the B-ultrasound operations. At present, the needle groove bodies of the existing structures, which are complicatedin installation and disassembly, cannot accomplish the purpose of simple operation, thereby increasing the difficulty of the doctor's operation during puncture operations.

The relevant prior art includes the patent application US 2007/078346 A1. In particular, as disclosed in the patent application US 2007/0078346 A1, paragraphs [0077] to [0078] and Figures 8A to 10, a needle clip in an ultrasonic guidance system used for guiding a needle of a probe can be obtained by the assembly of two parts. One of the two parts (the second part 202) is provided with a semi-circular cradle 204 for receiving the shaft of a needle, and can be engaged with another part (the first part 201) through sliding into a holding portion 226 of the first part 201. The semi-circular cradle 204 is of a fixed specification corresponding to a specified needle, and cannot be adjusted in shape and dimension. Such needle clip cannot accomplish the purpose of simple operation.

### Summary of the Invention

In order to overcome those technical defects in the prior art, i.e., complicated installation and disassembly of the needle groove body and the puncture frame body and an increased difficulty of the doctor's operation during puncture operations, the present invention provides a puncture frame according to claim 1. The puncture frame comprises one or more needle groove bodies and a puncture frame body. The needle groove body is simple in structure and is convenient in installation and disassembly. The doctor may operate the needle groove bodies and a puncture frame body with one hand, thereby reducing the difficulty of the doctor's operation during puncture operations.

The needle groove body, which has a simple structure, can facilitate its installation and disassembly with and from a mating puncture frame body.

Preferably, a boss is provided in a central portion at one end of the first fixing plate.

Preferably, the first fixing plate is provided with u-shaped grooves at positions on both sides of the boss.

Preferably, the second fixing plate is provided with two n-shaped engaging grooves.

A puncture frame body for use together with a needle groove body according to any one of the above is further disclosed. The puncture frame body has a connecting portion at one end and a sleeve-shaped portion at the other end, and the connecting portion is provided with the second groove plate and the inclined mounting plate are provided on the connection portion and are perpendicular to each other, and cooperate with the first fixing plate and the second fixing plate of the needle groove body, respectively, wherein the inclined mounting plate is provided with a first rib and a second rib which are parallel to each other, when the puncture frame body is used together with the needle groove body, the inclined mounting plate is fitted with the bottom of the needle groove body, the needle groove body can be sleeved outside the first rib and the second rib, and during the puncture operation, an ultrasonic probe can be inserted into and fixed with the sleeve-shaped portion such that the puncture frame body can be fixed to the ultrasonic probe.

Preferably, two hooks are provided at a top end on one side of the first rib away from the second rib, for catching the needle groove body.

Preferably, a reinforcing rib is provided at a bottom end on one side of the first rib close to the second rib, so as to enhance elastic feel in the hand.

Preferably, two limiting ribs are provided at a bottom end on one side of the second rib away from the first rib. The limiting ribs, which cooperate with the n-shaped engaging grooves on the needle groove body, can catch the needle groove body so that the needle groove body does not shake.

The disclosed puncture frame comprises one or more needle groove bodies according to any one of the above and a puncture frame body according to any one of the above.

Preferably, there are a plurality of needle groove bodies, and the first groove plates of the plurality of needle groove bodies are different in thickness.When the puncture frame body is used in cooperation with needle groove bodies of different specifications, the spacing between the first groove plate and the second groove plate would be changed to adapt to puncture needles of different specifications.

The present invention has the following advantageous effects:
1. The needle groove body has multiple different specifications and can adapt to puncture needles of different specifications in puncture operations;
2. the needle groove body is easy and convenient to fix and replace, easy to operate, and capable of allowing the doctor to operate with one hand;
3. a u-shaped needle groove can be formed between the first groove plate of the needle groove body and the second groove plate as well as the inclined mounting plate of the puncture frame body so as to accommodate free movement of apuncture needle in the u-shaped needle groove; and
4. the u-shaped needle groove, which is semi-open, can implement a function of needle-frame separation (separation of the puncture needle from the puncture frame) in puncture operations at any time.

### Brief Description of the Drawings

Fig. 1 is a structural schematic diagramof a needle groove body according to an embodiment of the present invention;
Figs. 2-1 to 2-3 are all views of the needle groove bodies viewed from the direction of a first fixing plate (which are needle groove bodies of three thickness specifications, respectively);
Fig. 3 is a structural schematic diagramof a puncture frame body according to an embodiment of the present invention;
Fig. 4 is a structural schematic diagramof a puncture frame according to an embodiment of the present invention;
Fig. 5 is an explosive view of the puncture frame according to the embodiment of the present invention.

In the figures, 1 -needle groove body; 11 - first groove plate; 12 - first fixing plate; 121 - boss; 122 - u-shaped groove; 123 - first indicator arrow; 124 - model mark; 13 - limiting plate; 14 - second fixing plate; 141 - n-shaped engaging groove; 2-connecting portion; 21 - second groove plate; 22 - inclined mounting plate; 23 - first rib; 231 -hook; 232 - reinforcing rib; 233 - second indicator arrow; 24 - second rib; 241 - limiting rib; 3 - u-shaped needle groove; 4 - sleeve-shaped portion; 5 - puncture frame body; 6 - puncture frame.

### Detailed Description of the Embodiments

Hereinafter, the structures of the present invention will be explained in detail with reference to the figures.

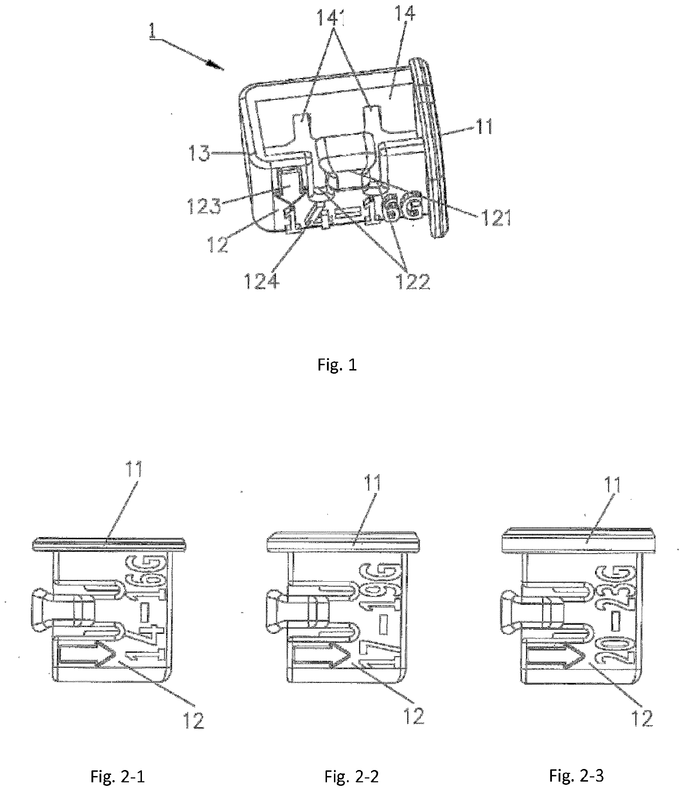

As shown in Fig. 1 and Figs. 2-1 to 2-3, a needle groove body 1 provided by an embodiment of the present invention is a hollow parallelepiped structure, and consists of a first groove plate 11, a first fixing plate 12, a limiting plate 13, and a second fixing plate 14 which are connected in sequence, wherein the first groove plate 11 can be designed in a variety of thickness specifications.

In the needle groove body 1 provided by the embodiment of the present invention, the first groove plate 11 and the limiting plate 13 are parallel to each other, and the first fixing plate 12 and the second fixing plate 14 are parallel to each other.

In the needle groove body 1 provided by the embodiment of the present invention, a boss 121 is providedin a central portion at one end of the first fixing plate 12.The first fixing plate 12 is provided with u-shaped grooves 122 at positions on both sides of the boss 121. The first fixing plate 12 is further provided with a first indicator arrow123 "↓" and a model mark124 for promptly indicating the specification of a needle in use. The second fixing plate 14is provided with two n-shaped engaging grooves 141.

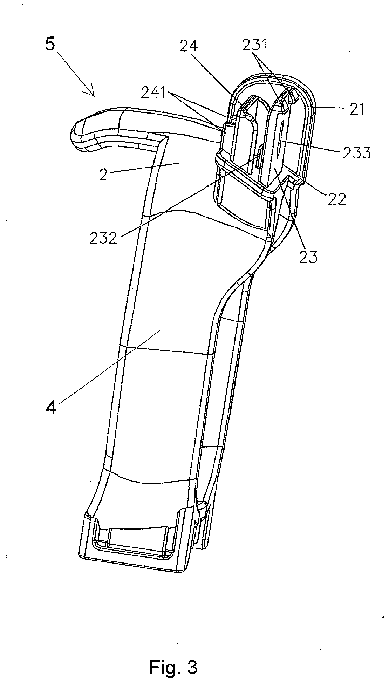

As shown in Fig. 3, a puncture frame body 5 for use together with the above needle groove body 1, as provided by an embodiment of the present invention, has a connecting portion 2at one end and a sleeve-shaped portion 4 at the other end, and the connecting portion 2 is provided with a second groove plate 21 and an inclined mounting plate 22 which are perpendicular to each other, wherein an upper surface of the inclined mounting plate 22is a needle insertion start plane, and the inclined mounting plate 22 can be fitted with the bottom of the needle groove body 1.

An ultrasonic probe can be inserted into and fixed with the sleeve-shaped portion 4 such that the puncture frame body 5 can be fixed to the ultrasonic probe (not illustrated).

In the puncture frame body 5 for use together with the above needle groove body 1, as provided by the embodiment of the present invention,the inclined mounting plate 22 is provided with a first rib 23 and a second rib 24 which are parallel to each other and cooperate with the first fixing plate 12 and the second fixing plate 14, respectively, wherein the needle groove body 1 can be sleeved outside the first rib 23 and the second rib 24.

In the puncture frame body 5 for use together with the above needle groove body 1, as provided by the embodiment of the present invention, two hooks 231 are provided at a top end on one side of the first rib 23 away from the second rib 24, for catching the needle groove body 1. A reinforcing rib 232 is provided at a bottom end on one side of the first rib 23 close to the second rib position 24, and can enhance elastic feel in the hand. A second indicator arrow233 "↓"is further provided in a central portion on one side of the first rib 23 away from the second rib 24.

In the puncture frame body for use together with the above needle groove body 1, as provided by the embodiment of the present invention, two limiting ribs 241, which are provided at a bottom end on one side of the second rib 24 away from the first rib 23, cooperate with the two n-shaped engaging grooves 141, respectively. The limiting ribs 241, which are engaged with the n-shaped engaging grooves 141 on the needle groove body 1, can catch the needle groove body 1 so that the needle groove body 1 does not shake.

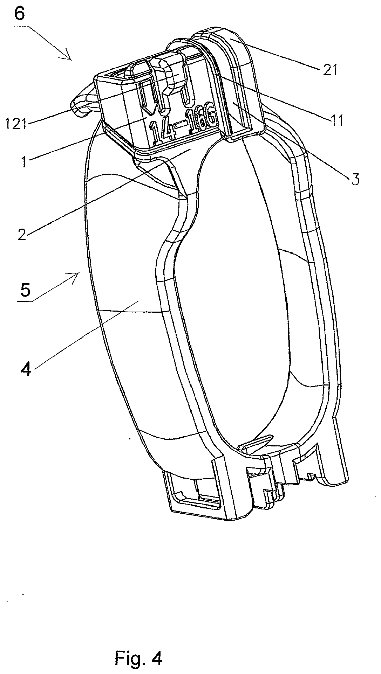

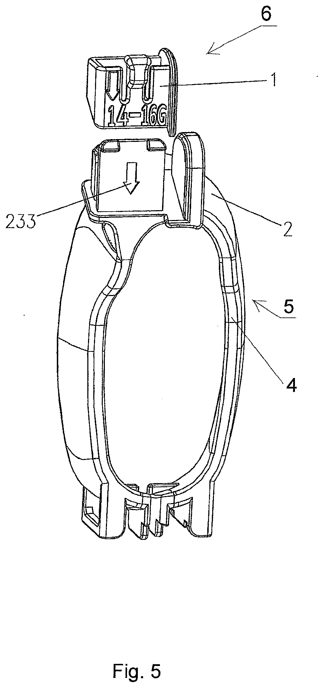

As shown in Figs. 4 to 5, a puncture frame 6 provided by an embodiment of the present invention comprises a plurality of the above needle groove bodies 1 and one of the above puncture frame bodies. The first groove plate 11 of the plurality of needle groove bodies 1 are different in thickness. When the puncture frame body is used in cooperation with needle groove bodies1 of different thickness specifications, the spacing between the first groove plate 11 and the second groove plate 21 would be changed to adapt to puncture needles of different specifications.

The puncture frame 6 provided by the embodiment of the present invention can be used in a method comprising the following operation steps: After the puncture frame body 5 is fixed to an ultrasonic B-ultrasound probe,the needle groove body 1 of a suitable specification is selected,and the side marked with the first indicator arrow 123 "↓" provided on the selected needle groove body 1 is placed in the same direction as the second indicator arrow 233 "↓" provided on the puncture frame body and ispressed down along a direction indicated by the second indicator arrow 233 "↓" on the puncture frame body. At this time, the two hooks 231 provided on the first rib 23 are deformed. When the needle groove body 1 is pushed into position (the bottom end of the needle groove body 1 is completely fitted with the inclined mounting plate 22), the two hooks 231 are elastically returned to clamp the needle groove body 1 on the puncture frame body for fixation, and after hearing a "snap" sound, this indicates that it has been installed in place. The two limiting ribs 241 provided on the second rib 24 are engaged with the two n-shaped engaging grooves 141 provided on the needle groove body 1 to catch the needle groove body 1 so that it does not shake.At this time, a free open u-shaped needle groove 3 is formed between the first groove plate 11 of the needle groove body 1 and the second groove plate 21 as well as the inclined mounting plate 22 of the puncture frame body, and, in a B-ultrasound puncture operation,the puncture needle can be freely inserted at any angle into the formed free open u-shaped needle groove 3. When the puncture needle specification needs to be changed during an operation, the structure of the boss 121provided on the needle groove body 1 is pinched and pressed inward until it stops, such that the two hooks 231 are separated from the needle groove body 1;the needle groove body 1 is lifted upward and taken out for a replacement with a needle groove body 1 of the required specification. The difference between the needle groove bodies 1 of different specificationslies in the wall thickness of the first groove plate 11 provided on the needle groove body 1, so that the spacing between the first groove plate 11 and the second groove plate 21 (i.e., the width of the u-shaped needle groove 3) can be adjusted, for the convenience of the operation.

The above are only preferred embodiments of the present invention and are not intended to limit the present invention. The scope of the invention is therefore defined by the wording of the claims.

## Claims

1. A puncture frame (6) comprising one or more needle groove bodies (1) and a puncture frame body (5),
the needle groove body (1) can be installed to a puncture frame body (5) to be fixed to an ultrasonic probe during a puncture operation,
**characterized in that** the needle groove body (1) comprises
a first groove plate (11),
a first fixing plate (12),
a limiting plate (13), and
a second fixing plate (14)
which are connected in sequence such that the needle groove body (1) is of a hollow parallelepiped structure and the needle groove body when placed on the puncture frame body, forms a u-shaped needle groove (3) suitable to guide the movement of a puncture needle, wherein the u-shaped needle groove (3) is formed by the first groove plate (11), a second groove plate (21) of the puncture frame body (5) and an inclined mounting plate (22) of the puncture frame body (5),
the puncture frame body (5) for use together with the needle groove body (1);
wherein the puncture frame body (5) has a connecting portion (2) at one end and a sleeve-shaped portion (4) at the other end, and the second groove plate (21) and the inclined mounting plate (22) are provided on the connecting portion (2) and are perpendicular to each other, wherein,
the inclined mounting plate (22) is provided with a first rib (23) and a second rib (24) which are parallel to each other and cooperate with the first fixing plate (12) and the second fixing plate (14) of the needle groove body (1), respectively,
when the puncture frame body (5) is used together with the needle groove body (1), the inclined mounting plate (22) is fitted with the bottom of the needle groove body (1), the needle groove body (1) can be sleeved outside the first rib (23) and the second rib (24),
and during the puncture operation, the ultrasonic probe can be inserted into and fixed with the sleeve-shaped portion such that the puncture frame body (5) can be fixed to the ultrasonic probe.

2. A puncture frame (6) according to claim 1, **characterized in that**, a boss (121) is provided in a central portion at one end of the first fixing plate (12).

3. A puncture frame (6) according to claim 2, **characterized in that**, the first fixing plate (12) is provided with u-shaped grooves (122) at positions on both sides of the boss (121).

4. A puncture frame (6) according to claim 1, **characterized in that**, the second fixing plate (14) is provided with two n-shaped engaging grooves (141).

5. A puncture frame (6) according to claim 1, **characterized in that**, two hooks (231) are provided at a top end on one side of the first rib (23) away from the second rib (24).

6. A puncture frame (6) according to claim 1, **characterized in that**, a reinforcing rib (232) is provided at a bottom end on one side of the first rib (23) close to the second rib (24).

7. A puncture frame (6) according to claim 1, **characterized in that**, two limiting ribs (241) are provided at a bottom end on one side of the second rib (24) away from the first rib (23).

8. The puncture frame (6) according to claim 1, **characterized in that**, there are a plurality of needle groove bodies (1), and the first groove plates (11) of the plurality of needle groove bodies (1) are different in thickness.

## Patentansprüche

1. Punktionsrahmen (6), der einen oder mehrere Nadelnutkörper (1) und einen Punktionsrahmenkörper (5) umfasst,
wobei der Nadelrillenkörper (1) an einem Punktionsrahmenkörper (5) installiert werden kann, um während eines Einstichvorgangs an einer Ultraschallsonde befestigt zu werden **dadurch gekennzeichnet, dass** der Nadelrillenkörper (1) umfasst
eine erste Rillenplatte (11),
eine erste Befestigungsplatte (12),
eine Begrenzungsplatte (13), und
eine zweite Befestigungsplatte (14) die nacheinander so verbunden sind, dass der Nadelrillenkörper (1) eine hohle Parallelepiped-Struktur aufweist und der Nadelrillenkörper, wenn er auf dem Punktionsrahmenkörper angeordnet ist, eine u-förmige Nadelrille (3) bildet, die geeignet ist, die Bewegung einer Punktionsnadel zu führen, wobei die u-förmige Nadelrille (3) durch die erste Rillenplatte (11), eine zweite Rillenplatte (21) des Punktionsrahmenkörpers (5) und eine geneigte Montageplatte (22) des Punktionsrahmenkörpers (5) gebildet wird, wobei der Punktionsrahmenkörper (5) zur Verwendung zusammen mit dem Nadelrillenkörper (1) dient; wobei der Punktionsrahmenkörper (5) einen Verbindungsabschnitt (2) an einem Ende und einen hülsenförmigen Abschnitt (4) an dem anderen Ende aufweist, und die zweite Rillenplatte (21) und die geneigte Montageplatte (22) an dem Verbindungsabschnitt (2) vorgesehen sind und senkrecht zueinander stehen, wobei,
die geneigte Montageplatte (22) mit einer ersten Rippe (23) und einer zweiten Rippe (24) versehen ist, die parallel zueinander verlaufen und mit der ersten Befestigungsplatte (12) bzw. der zweiten Befestigungsplatte (14) des Nadelrillenkörpers (1) zusammenwirken,
wenn der Punktionsrahmenkörper (5) zusammen mit dem Nadelfalzkörper (1) verwendet wird, die geneigte Befestigungsplatte (22) mit dem Boden des Nadelfalzkörpers (1) zusammengefügt wird, der Nadelfalzkörper (1) außerhalb der ersten Rippe (23) und der zweiten Rippe (24) ummantelt werden kann, und
während des Einstichvorgangs kann die Ultraschallsonde in den hülsenförmigen Abschnitt eingeführt und mit diesem befestigt werden, so dass der Punktionsrahmenkörper (5) an der Ultraschallsonde befestigt werden kann.

2. Punktionsrahmen (6) nach Anspruch 1, **dadurch gekennzeichnet, dass** ein Vorsprung (121) in einem zentralen Abschnitt an einem Ende der ersten Befestigungsplatte (12) vorgesehen ist.

3. Punktionsrahmen (6) nach Anspruch 2, **dadurch gekennzeichnet, dass** die erste Befestigungsplatte (12) mit u-förmigen Rillen (122) an Positionen auf beiden Seiten des Vorsprungs (121) versehen ist.

4. Punktionsrahmen (6) nach Anspruch 1, **dadurch gekennzeichnet, dass** die zweite Befestigungsplatte (14) mit zwei n-förmigen Eingriffsrillen (141) versehen ist.

5. Punktionsrahmen (6) nach Anspruch 1, **dadurch gekennzeichnet, dass** zwei Haken (231) an einem oberen Ende auf einer Seite der ersten Rippe (23) entfernt von der zweiten Rippe (24) vorgesehen sind.

6. Punktionsrahmen (6) nach Anspruch 1, **dadurch gekennzeichnet, dass** eine Verstärkungsrippe (232) an einem unteren Ende auf einer Seite der ersten Rippe (23) nahe der zweiten Rippe (24) vorgesehen ist.

7. Punktionsrahmen (6) nach Anspruch 1, **dadurch gekennzeichnet, dass** zwei Begrenzungsrippen (241) an einem unteren Ende auf einer Seite der zweiten Rippe (24) entfernt von der ersten Rippe (23) vorgesehen sind.

8. Punktionsrahmen (6) nach Anspruch 1, **dadurch gekennzeichnet, dass** eine Vielzahl von Nadelrillenkörpern (1) vorhanden ist und die ersten Rillenplatten (11) der Vielzahl von Nadelrillenkörpern (1) eine unterschiedliche Dicke aufweisen.

## Revendications

1. Cadre de perforation (6), comprenant un ou plusieurs corps de rainure d'aiguille (1) et un corps de cadre de perforation (5),
le corps de rainure d'aiguille (1) peut être installé sur un corps de cadre de perforation (5) pour être fixé à une sonde ultrasonique pendant une opération de perforation,
**caractérisé par le fait que** le corps de la rainure d'aiguille (1) comprend une première plaque de rainurage (11),
une première plaque de fixation (12),
une plaque de limitation (13), et
une deuxième plaque de fixation (14)
qui sont reliées l'une après l'autre de sorte que le corps de la rainure de l'aiguille (1) présente une structure parallélépipédique creuse et que le corps de la rainure de l'aiguille, lorsqu'il est placé sur le corps du cadre de perforation, forme une rainure d'aiguille en forme de u (3) apte à guider le mouvement d'une aiguille de perforation, la rainure d'aiguille en forme de u (3) est formée par la première plaque de rainure (11), une deuxième plaque de rainure (21) du corps de cadre de perforation (5) et une plaque de montage inclinée (22) du corps de cadre de perforation (5), le corps de cadre de perforation (5) étant destiné à être utilisé avec le corps de rainure d'aiguille (1);
dans lequel le corps du cadre de perforation (5) a une partie de connexion (2) à une extrémité et une partie en forme de manchon (4) à l'autre extrémité, et la deuxième plaque de rainure (21) et la plaque de montage inclinée (22) sont fournies sur la partie de connexion (2) et sont perpendiculaires l'une à l'autre, dans lequel,
la plaque de montage inclinée (22) est pourvue d'une première nervure (23) et d'une deuxième nervure (24) qui sont parallèles l'une à l'autre et coopèrent avec la première plaque de fixation (12) et la deuxième plaque de fixation (14) du corps de la rainure d'aiguille (1), respectivement, lorsque le cadre de perforation (5) est utilisé avec le corps de l'aiguille (1), la plaque de montage inclinée (22) est fixée au fond du corps de l'aiguille (1), le corps de l'aiguille (1) peut être manchonné à l'extérieur de la première nervure (23) et de la seconde nervure (24),
et pendant l'opération de perforation, la sonde à ultrasons peut être insérée et fixée à la partie en forme de manchon de sorte que le corps du cadre de perforation (5) puisse être fixé à la sonde à ultrasons.

2. Cadre de perforation (6) selon la revendication 1, **caractérisé en ce qu'**un bossage (121) est prévu dans une partie centrale à une extrémité de la première plaque de fixation (12).

3. Cadre de perforation (6) selon la revendication 2, **caractérisé en ce que** la première plaque de fixation (12) est pourvue de rainures en forme de u (122) à des positions situées de part et d'autre du bossage (121).

4. Cadre de perforation (6) selon la revendication 1, **caractérisé en ce que** la deuxième plaque de fixation (14) est pourvue de deux rainures d'engagement en forme de n (141).

5. Cadre de perforation (6) selon la revendication 1, **caractérisé en ce que** deux crochets (231) sont prévus à une extrémité supérieure sur un côté de la première nervure (23) à l'écart de la deuxième nervure (24).

6. Cadre de perforation (6) selon la revendication 1, **caractérisé en ce qu'**une nervure de renforcement (232) est prévue à une extrémité inférieure d'un côté de la première nervure (23) à proximité de la deuxième nervure (24).

7. Cadre de perforation (6) selon la revendication 1, **caractérisé en ce que** deux nervures de limitation (241) sont prévues à une extrémité inférieure d'un côté de la deuxième nervure (24) à l'écart de la première nervure (23).

8. Cadre de perforation (6) selon la revendication 1, **caractérisé par le fait qu'**il existe plusieurs corps de rainure d'aiguille (1) et que les premières plaques de rainure (11) de plusieurs corps de rainure d'aiguille (1) sont d'épaisseur différente.
